# EUROPÄISCHE PATENTANMELDUNG

(11) **EP 0 666 311 A1**
(43) Veröffentlichungstag der Anmeldung: **09.08.1995**
(21) Anmeldenummer: 95101594.0
(22) Anmeldetag: 06.02.1995
(51) Int. Cl.: C12M 1/12, C12M 1/04, C02F 3/20

(54) **Verfahren und Anlage zur blasenfreien Begasung von Mikroorganismen**

(30) Priorität: 07.02.1994 DE 4404600
(71) Anmelder: Ripperger, Siegfried, Prof. Dr., D-67724 Gonbach (DE)
(72) Erfinder: Ripperger, Siegfried, Prof. Dr., D-67724 Gonbach (DE)
(74) Vertreter: Vonnemann, Gerhard, Dr.-Ing.

(57) **Zusammenfassung**

Die Erfindung betrifft ein Verfahren sowie eine Anlage zur blasenfreien Begasung von Mikroorganismen, die in einem Reaktor auf einem Trägermaterial immobilisiert sind, wobei das Trägermaterial mit den Mikroorganismen von einer wäßrigen Lösung angeströmt wird, die vor dem Trägermaterial über Membranen, welche auf einer Seite mit einem sauerstoffhaltigen Gas beaufschlagt werden, mit Sauerstoff angereichert wurde.

## Beschreibung

Die Erfindung betrifft ein Verfahren und eine Anlage zur blasenfreien Begasung von Mikroorganismen, die in einem Reaktor auf einem Trägermaterial immobilisiert sind.

Die Verwendung von immobilisierten Mikroorganismen, die z.B. auf einem Trägermaterial wachsen, bietet Vorteile, wie z.B. eine einfache Handhabung und hohe Umsatzraten. Bioreaktoren mit immobilisierten Organismen werden daher bereits großtechnisch genutzt. Problematisch dabei sind aerobe Prozesse, da die Sauerstoffversorgung der Organismen schwierig ist. In der Regel wird die Flüssigkeit im Reaktor oder außerhalb durch einen zugeführten Luftstrom unter Bildung von Blasen begast. Dabei entsteht zwangsläufig ein Abluftstrom durch den gelöste Inhaltsstoffe der Flüssigkeit desorbiert und ausgetragen werden. Oft wird dadurch ein Abluftproblem verursacht, das durch zusätzliche Maßnahmen, wie z.B. durch den Betrieb von Abluftreinigungsverfahren, gelöst werden muß. Insbesondere bei biologischen Verfahren zur Abwasserreinigung muß mit dieser Problematik gerechnet werden.

Bei dem erfindungsgemäßen Verfahren wird das Problem durch den Einsatz von Membranen zur blasenfreien Begasung der flüssigen Phase gelöst. Das Trägermaterial mit den immobilisierten Organismen wird ständig von einer wäßrigen Lösung überströmt, die zuvor in einer Membraneinheit mit Sauerstoff beladen wurde.

Die blasenfreie Begasung von Flüssigkeiten über Membranen ist bereits bekannt. In DE 31 07 874 A1 wird die Möglichkeit des Einsatzes von mikroporösen Membranen beschrieben. J. Lehmann et al beschreiben in Biotech-Forum 2 (1985) Nr. 3, S. 112-118 die Anwendung von bewegten mikroporösen Membranen zur blasenfreien Zellkulturbegasung in einem Fermenter. P.A. Wilderer berichtet in Conservation & Recycling Vol. 8 (1985), Nos. 1/2, pp. 181-192 über den Einsatz von homogenen, dichten Membranen aus Silikon zur blasenfreien Begasung von Reaktoren zur Abwasserbehandlung. Bei den bekannten Fällen handelte es sich um submerse Fermentationen, bei denen die Membran innerhalb des Fermenter in der Fermenterbrühe eingebaut waren. Es werden hierzu oft komplizierte Membraneinheiten verwendet die zur Verbesserung des Stoffübergangs und der Vermischung im Fermenter bewegt werden.

Aufgabe der Erfindung ist es, einen Fermenter, der verringerte Abluftprobleme aufweist und mit geringerem Aufwand herzustellen ist.

Diese Aufgabe wird dadurch gelöst, daß das Trägermaterial mit den Mikroorganismen von einer wäßrigen Lösung angeströmt wird, die vor dem Trägermaterial über Membranen, welche auf einer Seite mit einem sauerstoffhaltigen Gas beaufschlagt werden, mit Sauerstoff angereichert wurde.

Zur Durchführung des Verfahrens ist eine Anlage gemäß den Merkmalen des Anspruchs 8 geeignet.

Die Nutzung einer Membraneinheit zur blasenfreien Begasung außerhalb eines Reaktors, in dem Mikroorganismen auf einem Trägermaterial, das z.B. als Festbett vorliegt, immobilisiert sind, erlaubt es vorteilhaft, hierzu handelsübliche Membraneinheiten, wie sie z.B. zur Mikrofiltration verwendet werden, einzusetzen. Überraschend ist, daß damit auch eine günstige Sauerstoffversorgung der immobilisierten Mikroorganismen erreicht werden kann.

Weitere vorteilhafte Ausgestaltungen sind in den Unteransprüchen 2 bis 7 für das Verfahren beschrieben und für die Anlage in den Unteransprüchen 9 bis 11.

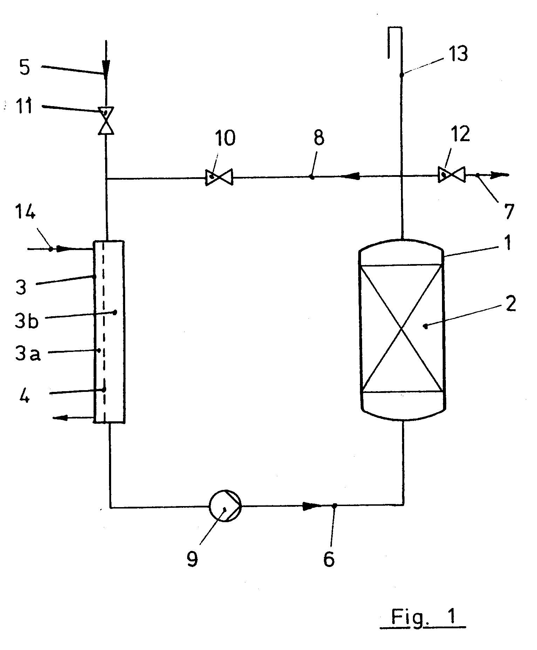

Die Erfindung ist in der Zeichnung Figur 1 in bevorzugter Ausführungsform beschrieben.
- Fig. 1: zeigt einen schematischen Aufbau der erfindungsgemäßen Vorrichtung.

Die in Fig. 1 dargestellte Vorrichtung weist eine Zuleitung 5 auf, welche die wäßrige Lösung über ein Ventil 11 einer Membraneinheit 3 zuführt. Die Membraneinheit wird durch die Membran 4 in zwei Kammern, eine flüssigkeitsseitige 3b, welche von der Lösung durchströmt wird und eine gasseitige 3a, welche von Luft oder Sauerstoff angereicherter Luft ausgefüllt wird, aufgeteilt. Die Zuleitung 5 führt zur flüssigkeitsseitigen Kammer 3b. Sauerstoff kann durch die Membran 4 in die Flüssigkeit diffundieren. Die mit Sauerstoff angereicherte Lösung wird danach über eine Leitung 6 dem Behälter 1 zugeführt, in dem das Trägermaterial mit den immobilisierten Mikroorganismen 2 enthalten ist. In der Leitung 6 kann eine Pumpe 9 zur Flüssigkeitsförderung eingebaut sein. Nach dem Behälter ist eine Ableitung 7 und ein Ventil 12 angeordnet, über die entsprechend der zulaufenden Flüssigkeitsmenge ein Abfluß stattfindet. Über die Leitung 8 kann ein Flüssigkeitsstrom rückgeführt werden, so daß eine Zirkulation der Lösung innerhalb der Anlage aufrechterhalten werden kann. Der zirkulierende Anteil kann über die Betriebsparameter der Pumpe 9 und das Ventil 10 variiert werden. Die Entlüftung 13 dient dazu das Druckniveau innerhalb der Anlage auf Atmosphärendruck zu halten und z.B. entstehenden CO₂ abzuleiten. Das sauerstoffhaltige Gas wird über die Leitung 14 der Membraneinheit zugeführt.

Als Trägermaterial für die Mikroorganismen kann z.B. Aktivkohle verwendet werden. Aktivkohle wird zur Adsorption von organischen Stoffen aus Flüssigkeiten und Gasen angewendet. Dabei handelt es sich überwiegend um Schadstoffe. Während bei Anwendungen von Aktivkohle zur Gasreinigung eine Regeneration, z.B. mit Wasserdampf oder einem warmen Trägergasstrom üblich ist, wird bei einem Einsatz in der wäßrigen Phase die Aktivkohle einmal verwendet. Nach einer Beladung mit organischen Stoffen wird sie entweder verbrannt oder durch aufwendige thermische Regenerationsverfahren, die oft dem thermischen Aktivierungsprozeß nachgebildet sind, reaktiviert. Die Reaktivierung findet meist nicht am Ort des Aktivkohleeinsatzes statt.

Es ist bekannt, daß Aktivkohle von Mikroorganismen besiedelt wird. Insbesondere bei der Trinkwasseraufarbeitung wird die Besiedlung in Verbindung mit der Adsorptionsfähigkeit der Kohle und dem mikrobiologischen Abbau von organischen Verbindungen zur Eliminierung von Schadstoffen genutzt. Durch eine blasenfreie Begasung der Flüssigkeit mit Luft, Sauerstoff oder Ozon kann dieser Abbau wesentlich gesteigert werden.

Mit dem erfindungsgemäßen Verfahren ist es auch möglich, mit Schadstoffen beladene Aktivkohle, die von Mikroorganismen besiedelt ist, umweltschonend zu regenerieren. Es kann schon von Vorteil sein, wenn nur einige Komponenten abgebaut werden, um die Adsorptionsfähigkeit für schwer abbaubare Komponenten dadurch zu erhöhen. In der Praxis kann auf diese Weise eine Standzeitverlängerung eines Aktivkohlefestbettes erreicht werden. Eine Voraussetzung für den biologischen Abbau der Stoffe unter aeroben Bedingungen ist die ausreichende Versorgung der Mikroorganismen mit Sauerstoff. Eine Begasung des Festbettes durch Einblasen von Luft kann bei den überwiegend auch vorhandenen flüchtigen Verbindungen zu einem Abluftproblem führen. Es wäre in solchen Fällen eine aufwendige Abluftreinigung vorzusehen, in der die gestrippten Schadstoffe aus dem Gasstrom abgetrennt werden.

Bei dem erfindungsgemäßen Verfahren tritt dieses Problem nicht auf. Bei dem Verfahren wird das Festbett ständig mit einem Sauerstoff beladenen Wasserstrom versorgt. Der Wasserstrom wird im Kreislauf geführt und in einer gesonderten Stufe über Membranen blasenfrei mit Sauerstoff versorgt. Auch hierzu kann der in Fig. 1 gezeigte schematische Aufbau der erfindungsgemäßen Vorrichtung verwendet werden. Die Vorrichtung wird über die Leitung 5 mit Wasser gefüllt. Danach wird das Ventil 11 geschlossen und die Flüssigkeit zirkuliert innerhalb der Vorrichtung, so daß ständig in der Membraneinheit 3 begaste Flüssigkeit dem Behälter 1, in dem das Trägermaterial mit den Mikroorganismen (in diesem Fall Aktivkohle) 2 enthalten ist, zugeführt wird.

Die Funktionsfähigkeit der in Fig. 1 dargestellten Begasungsvorrichtung konnte durch Versuche nachgewiesen werden. Hierzu wurde ein Festbettreaktor, in dem sich eine Aktivkohle befand, die mit einem Gemisch aus organischen Stoffen eines Abwasser beladen wurde, ständig mit umgewälztem Wasser durchströmt. Der Festbettreaktor hatte eine Höhe von 23 cm und einen Innendurchmesser von 7 cm. Es wurden 383 g einer Feuchtkohle eingewogen. Es war bekannt, daß die Aktivkohle mit Mikroorganismen besiedelt war. Zusätzlich wurden sie mit einer Mischkultur beimpft. Die Membranen lagen in Kapillarform vor (Innendurchmesser 1,8 mm, Außendurchmesser 2,6 mm). Insgesamt wurden 40 Kapillaren mit einer Länge von 500mm in Form eines Kapillarmoduls eingesetzt. Die Membranstruktur war mikroporös und wies eine Porosität von ca. 75% und einen maximalen Porendurchmesser < 0,9 µm auf. Der maximale Porendurchmesser wurde mit der Blaspunktdruckmethode ermittelt. Das Membranmaterial war Polypropylen. Zur Umwälzung der Flüssigkeit wurde eine kleine Kreiselpumpe (Fabrikat Comet, Kriftel) verwendet. In die einzelnen Apparateteile wurden 1,25 l destilliertes Wasser eingefüllt. Nach dem Befüllen der Anlage wurde mit der Kreiselpumpe ein Volumenstrom von 12,5 ml/min ± 2,5 ml/min eingestellt. Die Wirksamkeit des Begasungssystems wurde mittels einer Sauerstoffbestimmung in der wäßrigen Lösung ermittelt. Hierzu wurde das Meßgerät Oxi 91 der Firma WTW, Weilheim, verwendet. Als Meßergebnis wurde der Sauerstoffgehalt in Prozent der möglichen Sättigungskonzentration angegeben. Die Temperatur wurde über die ganze Versuchsserie nahezu konstant gehalten (21 °C ± 1 ° C).

Die Anlage wurde zunächst über drei Wochen mit dem eingebauten Membranmodul betrieben. In dieser Zeit fiel die O₂-Konzentration von anfänglich 87 % auf 55 % ab, wobei in der ersten Woche bereits ein Abfall auf 65 % zu verzeichnen war. Es kann angenommen werden, daß die Mikroorganismenpopulationen sich in dieser Zeit an die Milieubedingungen angepaßt haben und der O₂-Gehalt von 55 % ein stationäres Gleichgewicht zwischen O₂-Zufuhr und O₂-Umsatz darstellt. Bei einem Abschalten der Kreiselpumpe sank der Sauerstoffgehalt infolge der fehlenden Umwälzung rapide auf 16 % ab. Ebenso wurde bei einer Überbrückung des Membranmoduls ein Abfall des Sauerstoffgehaltes auf Werte unter 20 % beobachtet. Nach solchen Manipulationen konnte durch Anfahren der Pumpe bzw. ein Zwischenschalten des Membranmoduls in 15 bis 30 Minuten wieder ein Sauerstoffgehalt zwischen 50 und 60 % erreicht werden. Diese Ergebnisse mach deutlich, daß eine Sauerstoffversorgung immobilisierter Mikroorganismen mit dem in Figur 1 aufgetragenen Begasungssystem möglich ist.

### BEZUGSZEICHENLISTE

- 1: Behälter
- 2: immobilisierte Mikroorganismen
- 3: Membraneinheit
- 3a: gasseitige Kammer
- 3b: flüssigkeitsseitige Kammer
- 4: Membran
- 5: Zuleitung
- 6: Leitung
- 7: Ableitung
- 8: Leitung
- 9: Pumpe
- 10: Ventil
- 11: Ventil
- 12: Ventil
- 13: Entlüftung
- 14: Leitung

## Patentansprüche

**1.** Verfahren zur blasenfreien Begasung von Mikroorganismen, die in einem Reaktor auf einem Trägermaterial immobilisiert sind, **dadurch gekennzeichnet**, daß das Trägermaterial mit den Mikroorganismen von einer wäßrigen Lösung angeströmt wird, die vor dem Trägermaterial über Membranen, welche auf einer Seite mit einem sauerstoffhaltigen Gas beaufschlagt werden, mit Sauerstoff angereichert wurde.

**2.** Verfahren nach Anspruch 1, **dadurch gekennzeichnet**, daß die wäßrige Lösung im Kreis geführt wird.

**3.** Verfahren nach Anspruch 1 oder 2, **dadurch gekennzeichnet**, daß es sich bei dem Trägermaterial, auf dem die Mikroorganismen wachsen, um Aktivkohle handelt.

**4.** Verfahren nach Anspruch 1, 2 oder 3, **dadurch gekennzeichnet**, daß das Trägermaterial ein Adsorbens ist und, daß adsorbierte Schadstoffe auf dem Adsorbens von den darauf wachsenden Mikroorganismen abgebaut werden und die Adsorptionsfähigkeit des Adsorbens dadurch wieder erhöht wird.

**5.** Verfahren nach Anspruch 1, 2, 3 oder 4, **dadurch gekennzeichnet**, daß die Begasung mit einer Membran betrieben wird, deren maximale Porengröße nicht größer als 0,9 µm ist und die eine Porosität von mindestens 40 % aufweist.

**8.** Anlage zur Durchführung des Verfahrens zur blasenfreien Begasung von Mikroorganismen, die in einem Reaktor auf einem Trägermaterial immobilisiert sind, **dadurch gekennzeichnet**, daß das Trägermaterial mit den Mikroorganismen in einer wäßrigen Lösung angeordnet ist, in der Membranen vorgesehen sind, welche auf einer Seite mit einem Sauerstoffhaltigen Gas beaufschlagt sind, um die Lösung mit Sauerstoff anzureichern.

**9.** Anlage nach Anspruch 8, **dadurch gekennzeichnet**, daß zwischen Membran und Trägermaterial ein möglichst geringer Abstand vorgesehen ist.

**10.** Anlage nach Anspruch 8 oder 9, **dadurch gekennzeichnet**, daß für die wäßrige Lösung ein Kreislauf vorgesehen ist.

**11.** Anlage nach Anspruch 8, 9 oder 10, **dadurch gekennzeichnet**, daß das Trägermaterial, Aktivkohle ist.
